# EUROPÄISCHE PATENTSCHRIFT

(11) **EP 2 103 934 B1**
(45) Veröffentlichungstag und Bekanntmachung des Hinweises auf die Patenterteilung: **17.04.2019**
(21) Anmeldenummer: 08153092.5
(22) Anmeldetag: 20.03.2008
(51) Int. Cl.: G01N 33/487, A61B 5/15

(54) **Verfahren zum Herstellen einer diagnostischen Bandeinheit**
Method for production of a diagnostic tape
Procédé de fabrication d'une unité de bande diagnostique

(43) Veröffentlichungstag der Anmeldung: 23.09.2009
(73) Patentinhaber: F. Hoffmann-La Roche AG, 4070 Basel (CH); Roche Diabetes Care GmbH, 68305 Mannheim (DE)
(72) Erfinder: Ditscher, Wolfgang, 67691 Hochspeyer (DE); Joest, Karlheinz, 69483 Wald-Michelbach (DE); Dambach, Ria, 68165 Mannheim (DE); Kasejew, Alexander, 68199 Mannheim (DE); Aigl, Daniel, 68535 Edingen-Neckarhausen (DE)
(74) Vertreter: Pfiz, Thomas

(56) Entgegenhaltungen:
- EP-A- 0 299 517
- EP-A- 0 974 964
- EP-A- 1 593 434
- EP-A1- 1 852 699
- EP-A2- 0 232 811
- WO-A-2007/077212
- WO-A1-2005/104948
- WO-A2-2005/107596
- DE-A1- 4 040 381
- DE-A1- 19 849 539
- US-A- 4 062 719
- US-A- 4 218 421
- US-A- 4 947 277

## Beschreibung

Die Erfindung betrifft ein Verfahren zur Herstellung einer diagnostischen Bandeinheit nach Anspruch 1. Weitere Ausführungsformen werden in den abhängigen Ansprüchen beschrieben.

Solche Bandeinheiten wurden vor allem für Blutzuckertests konzipiert, um gegenüber den am Markt befindlichen Teststreifensystemen die Benutzerfreundlichkeit weiter zu verbessern. So kann im Sinne einer vereinfachten Handhabung auf einem aufrollbaren Transportband eine hohe Anzahl von Testeinheiten kompakt bevorratet und durch den Bandtransport nach Gebrauch auch wieder entsorgt werden. Zweckmäßig soll eine solche Bandeinheit in Form einer Kassette als Verbrauchsmaterial in ein Handgerät einsetzbar sein, um dem Benutzer weitgehend automatisch ablaufende Selbsttests zu ermöglichen. Bisherige Veröffentlichungen, wie etwa die WO 2005/104948, befassen sich vor allem mit der vereinfachten Integration der Testeinheiten auf einem Trägerband.

EP299517 beschreibt in Spalten 45 und 46 die Herstellung einer diagnostischen Bandeinheit. Dabei wird ein Vorläuferband unabhängig vom Testband an einer Vorläuferspule befestigt. In einem späteren Herstellungsschritt werden diese beiden Bänder dann miteinander verbunden.

DE19849539 beschreibt einen Glucose-Walkman mit einer Cassette. Die Struktur der Cassette ist wie von Musikcassetten bekannt.

Ausgehend hiervon liegt der Erfindung die Aufgabe zugrunde, die im Stand der Technik bekannten Verfahren und Erzeugnisse weiter zu entwickeln und eine auch hinsichtlich der Massenfertigung optimierte Bauweise für eine zuverlässig arbeitende Bandeinheit der eingangs angegebenen Art anzugeben.

Zur Lösung dieser Aufgabe wird die in dem unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Dementsprechend wird in verfahrensmäßiger Hinsicht vorgeschlagen, dass das Transportband über ein Hilfsband an seinem vorlaufenden Ende mit der Aufwickelspule und/oder an seinem nachlaufenden Ende mit der Abwickelspule verbunden wird. Die Bandbefestigung an den Spulen kann auf diese Weise vereinfacht werden und muss nicht in den Wickelprozess des Testband- bzw. Gutmaterials integriert werden. Insbesondere muss auch nicht mehr Gutmaterial als für die Tests benötigt aufgewickelt werden. So kann auch die letzte Testeinheit noch probrung z.B. hinsichtlich der Testverteilung des Gutmaterials verzichtet werden. Ein weiterer Vorteil liegt darin, dass das Hilfsband für die Spulenverbindung vor allem in kurzen Zykluszeiten optimiert und in weiteren Fertigungsschritten beispielsweise für Drehmomentprüfungen an den Spulen genutzt werden kann, ohne dafür Gutband zu verwenden.

Weiterhin sieht das **erfindungsgemäße** Verfahren vor, dass die Aufwickelspule und die Abwickelspule zunächst über das Hilfsband verbunden werden, und dass sodann das Hilfsband zwischen den Spulen unter Bildung eines Vorläuferbands und Nachläuferbands aufgetrennt und mit den Bandenden des Transportbands verbunden wird.

Im erfindungsgemäßen Verfahren werden die
Testeinheiten auf Ausschuss untersucht und ein entsprechender, Schlechtmaterial enthaltender Teil des Transportbands ausgeschnitten, und sodann die verbleibenden Transportbandabschnitte an einer Verbindungsstelle mittels eines Klebebandstücks wieder zusammengesetzt.

Um eine einfache Bandverbindung ohne offenen Klebespalt zu schaffen, ist es vorteilhaft, wenn das Transportband über ein Klebebandstück Stoß auf Stoß mit dem Hilfsband verbunden wird.

Alternativ ist es auch möglich, dass das Transportband überlappend mit dem Hilfsband verbunden wird. Hierfür ist es günstig, wenn das Transportband und das Hilfsband in einem endseitigen Überlappbereich stoffschlüssig miteinander verbunden, vorzugsweise verklebt werden.

Ein weiterer besonderer Aspekt der Erfindung liegt darin, dass das Transportband über ein damit verbundenes Hilfsband an einem freien Bandendabschnitt über eine stoffschlüssige oder kraftschlüssige Bandverbindung mit der Abwickelspule und/oder Aufwickelspule fest verbunden wird, so dass sich der Bandendabschnitt beim Abspulen nicht selbständig löst. Durch ein solches Verbindungsprinzip lässt sich die Montage weiter vereinfachen, und es können kompakte Spulen ohne zusätzliche Hilfsmittel oder Teile geschaffen werden.

Vorteilhafterweise wird die stoffschlüssige Bandverbindung durch Schweißen oder Kleben hergestellt. Dies lässt sich besonders einfach dadurch bewerkstelligen, dass die stoffschlüssige Bandverbindung durch ein Heißklebemittel hergestellt wird. Hierfür können in einem vorbereitenden Schritt der Bandendabschnitt und/oder die damit zu verbindende Spule als Verbindungspartner mit einem Heißklebemittel beschichtet werden, wobei sich nachfolgend die Verbindungspartner durch Anwendung von Druck und Wärme vorzugsweise mittels eines Schweißstempels flächig miteinander verbunden lassen.

Eine weitere Verbesserung sieht vor, dass das Heißklebemittel nass aufgebracht und anschließend getrocknet wird, so dass ein Feststoffanteil als Klebeschicht verbleibt. Bevorzugt wird eine Klebeschicht mit einer Schichtdicke von weniger als 50 µm, vorzugsweise etwa 10 µm aufgebracht.

Herstellungstechnisch ist es auch von Vorteil, wenn das Hilfsband vorzugsweise durch ein Koextrusionsverfahren über seine Länge mit Heißklebemittel beschichtet wird.

Für eine sichere Befestigung hat es sich als hinreichend erwiesen, wenn der Bandendabschnitt über einen Umschlingungsbereich von weniger als 180°, vorzugsweise weniger als 90° fest mit der Spule verbunden wird.

Denkbar ist es auch, dass die kraftschlüssige Bandverbindung durch eine Klemmverbindung zwischen dem Bandendabschnitt und der zugeordneten Spule hergestellt wird. Hierbei lässt sich der Teileaufwand weiter verringern, indem der Bandendabschnitt in einem Klemmspalt der zugeordneten Spule eingeklemmt wird.

Eine weitere Variante der Erfindung sieht vor, dass das Transportband aus zwei an einer Verbindungsstelle zusammengefügten Transportbandabschnitten gebildet wird. Eine solche Spleißverbindung erlaubt die gezielte Strukturierung von Abschnitten eines als Endlosmaterial hergestellten Testbands. Hierbei wird gemäß einer bevorzugten Ausgestaltung in einem ersten Schritt ein als Ausschuss erfasster Teil des Transportbands ausgeschnitten, und anschließend werden die verbleibenden Transportbandabschnitte zusammengespleißt.

Vorteilhafterweise werden die Transportbandabschnitte über ein Klebebandstück an der Verbindungsstelle miteinander verbunden, wobei es besonders günstig ist, wenn die Transportbandabschnitte an der Verbindungsstelle Stoß auf Stoß miteinander verbunden werden.

Im Folgenden wird die Erfindung anhand der in der Zeichnung schematisch dargestellten Ausführungsbeispiele näher erläutert. Es zeigen:
- Fig. 1: eine diagnostische Bandeinheit in Form einer Bandkassette in einem vereinfachten Querschnitt;
- Fig. 2: eine Vorrichtung zur Vorkonfektionierung der Bandeinheit nach Fig. 1;
- Fig. 3 und 4: eine Bandanordnung der Bandeinheit mit klebenden Bandhaltemitteln zur Verbindung mit den Wickelspulen.

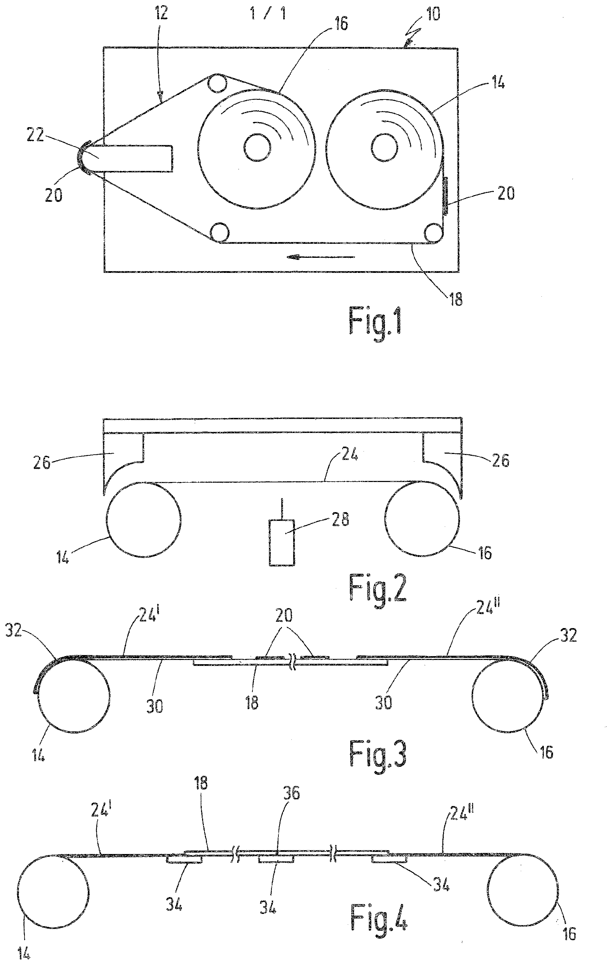

Die in Fig. 1 dargestellte diagnostische Bandkassette 10 zur Durchführung von Blutzuckertests umfasst ein Testband 12, eine Abwickelspule 14 zum Abwickeln von ungebrauchtem Testband und eine Aufwickelspule 16 zum Aufwickeln von gebrauchtem Testband, wobei das Testband 12 ein aufrollbares Transport- bzw. Trägerband 18 und eine Vielzahl von darauf für einen sukzessiven Einmalgebrauch bevorrateten Testeinheiten 20 aufweist. Die dargestellten Testeinheiten 20 sind als Testfelder mit einer Testchemie für einen Glucosenachweis versehen. Alternativ oder ergänzend können auch nicht gezeigte Stechelemente für einen Hauteinstich auf dem Transportband 18 bereitgestellt werden. Das Testband 12 lässt sich über einen Drehantrieb der Aufwickelspule 16 vorspulen, so dass die Testeinheiten 20 an einer Applikationsstelle 22 für den Benutzer bereitstellbar sind. Zweckmäßig lässt sich eine solche Bandkassette 10 in einem nicht gezeigten Handgerät als Verbrauchsmaterial für Benutzer-Selbsttests einsetzen. Weitere Details einer solchen Anwendung ergeben sich beispielsweise aus der Anmeldung EP-A 1 878 379, auf die in diesem Zusammenhang Bezug genommen wird.

Fig. 2 veranschaulicht einen Produktionsschritt für die Vorkonfektionierung der Bandkassette 10. Zur Bandbefestigung wird zunächst ein Hilfsband 24 an seinen Bandenden stoffschlüssig mit der Aufwickelspule 14 und der Abwickelspule 16 verbunden. Dies kann durch eine Heißklebeverbindung mittels Schweißstempeln 26 erfolgen, die gegen ein Spulensegment anpreßbar sind, um das Heißklebemittel zu aktivieren. Sodann wird das Hilfsband 24 durch eine Schneidevorrichtung 28 aufgetrennt, um das mit den Testeinheiten 20 versehene Transportband 18 in gewünschter Länge anzuspleißen.

In dem in Fig. 3 gezeigten Beispiel ist das Hilfsband 24 mit einer Heißklebeschicht 30 beschichtet. Hierfür wird ein Heißklebemittel durch ein Koextrusionsverfahren bei der Herstellung des Hilfsbandes einseitig flüssig aufgebracht und getrocknet, so dass ein Feststoffanteil als Heißklebeschicht 30 mit einer Schichtdicke von etwa 10 µm verbleibt. Beim anschließenden Verkleben mittels der Schweißstempel 26 wird dann eine Bandverbindung 32 auf den Spulen 14, 16 geschaffen, die das Hilfsband 24 gegen selbsttätiges Lösen sichert.

In einem weiteren Fertigungsschritt wird das in ein mit der Abwickelspule 14 verbundenes Nachläuferband 24' und ein mit der Aufwickelspule 16 verbundenes Vorläuferband 24" zerteilte Hilfsband mit dem Transportband 18 verbunden. Das in Fig. 3 unterbrochen gezeigte Transportband 18 kann dabei von einem Endlosmaterial in geeigneter Länge abgelängt und eingespleißt werden, um eine gewünschte Anzahl von Testeinheiten 20 bereitzustellen. Zu diesem Zweck werden die freien Bandenden überlappend zusammengefügt und unter Aktivierung der Heißklebeschicht 30 in dem Überlappbereich 30 fest verklebt. Im unbenutzten Zustand in der Kassette 10 ist das Bandmaterial 12 auf die Aufwickelspule 14 aufgewickelt, und der Bandabzug erfolgt zunächst über das Vorläuferband 24", bis die erste Testeinheit 20 bereitsteht.

Fig. 4 zeigt ein weiteres Beispiel, bei dem eine Bandverbindung über gesonderte Klebebandstücke 34 geschaffen wird. Hierbei werden das Nachläuferband 24' und das Vorläuferband 24" überlappungsfrei Stoß auf Stoß zusammengefügt, wobei die Klebebandstücke 30 die im Gebrauch auftretenden Zugbeanspruchungen aufnehmen.

Gemäß der Erfindung wird eine solche Klebebandverbindung auch eingesetzt, um Schlechtmaterial aus dem Testband 12 auszuschleusen. Zu diesem Zweck werden die Testeinheiten 20 auf Ausschuss untersucht und ein entsprechender Teil des Transportbands 18 ausgeschnitten. Sodann können die verbleibenden Transportbandabschnitte an einer Verbindungsstelle 36 mittels eines Klebebandstücks 34 wieder zusammengesetzt werden.

Die Verbindung des Hilfsbands 24 mit den Spulen 14, 16 kann auch klebstofffrei erfolgen, beispielsweise durch Ultraschallschweißen oder durch eine Klemmverbindung, indem das Bandende in einem Klemmspalt der betreffenden Spule kraftschlüssig gehalten wird.

## Patentansprüche

1. Verfahren zum Herstellen einer diagnostischen Bandkassette (10) zur Durchführung von Blutzuckertests, (10) bei welchem auf einem aufrollbaren Transportband (18) als Analyseelemente ausgebildete disposible Testeinheiten (20) für Blutzuckertests aufgebracht werden, und bei welchem das Transportband (18) auf eine Abwickelspule (14) gewickelt und mit einer Aufwickelspule (16) verbunden wird, so dass die Testeinheiten (20) für einen Benutzer durch Vorspulen des Transportbands (18) sukzessive bereitstellbar sind,
und bei dem das Transportband (18) über ein Hilfsband (24) an seinem vorlaufenden Ende mit der Aufwickelspule (16) verbunden wird,
**dadurch gekennzeichnet, dass** die Testeinheiten (20) auf Ausschuss untersucht und ein entsprechender, Schlechtmaterial enthaltender Teil des Transportbands (18) ausgeschnitten wird, und sodann die verbleibenden Transportbandabschnitte an einer Verbindungsstelle (36) mittels eines Klebebandstücks (34) wieder zusammengesetzt werden, und dass das Transportband (18) über das Hilfsband (24) an seinem nachlaufenden Ende mit der Abwickelspule (14) verbunden wird.

2. Verfahren nach Anspruch 1, **dadurch gekennzeichnet, dass** das Transportband (18) über ein Klebebandstück (34) Stoß auf Stoß mit dem Hilfsband (24) verbunden wird.

3. Verfahren nach Anspruch 1, **dadurch gekennzeichnet, dass** das Transportband (18) überlappend mit dem Hilfsband (24) verbunden wird.

4. Verfahren nach Anspruch 3, **dadurch gekennzeichnet, dass** das Transportband (18) und das Hilfsband (24) in einem endseitigen Überlappbereich stoffschlüssig miteinander verbunden, vorzugsweise über eine Heißklebeschicht (30) des Hilfsbands (24) verklebt werden.

5. Verfahren nach einem der Ansprüche 1 bis 4, **dadurch gekennzeichnet, dass** das Transportband (18) oder ein damit verbundenes Hilfsband (24) an einem freien Bandendabschnitt über eine stoffschlüssige oder kraftschlüssige Bandverbindung (32) mit der Abwickelspule (14) und/oder Aufwickelspule (16) fest verbunden wird, so dass sich der Bandendabschnitt beim Abspulen nicht selbständig löst.

6. Verfahren nach Anspruch 5, **dadurch gekennzeichnet, dass** die stoffschlüssige Bandverbindung (32) durch Schweißen oder Kleben hergestellt wird.

7. Verfahren nach Anspruch 5 oder 6, **dadurch gekennzeichnet, dass** die stoffschlüssige Bandverbindung (32) durch ein Heißklebemittel hergestellt wird.

8. Verfahren nach einem der Ansprüche 5 bis 7, **dadurch gekennzeichnet, dass** in einem vorbereitenden Schritt der Bandendabschnitt und/oder die damit zu verbindende Spule als Verbindungspartner mit einem Heißklebemittel beschichtet werden, und dass nachfolgend die Verbindungspartner durch Anwendung von Druck und Wärme vorzugsweise mittels eines Schweißstempels flächig miteinander verbunden werden.

9. Verfahren nach einem der Ansprüche 5 bis 8, **dadurch gekennzeichnet, dass** das Heißklebemittel nass aufgebracht und anschließend getrocknet wird, so dass ein Feststoffanteil als Klebeschicht (30) verbleibt.

10. Verfahren nach einem der Ansprüche 5 bis 9, **dadurch gekennzeichnet, dass** eine Klebeschicht (30) mit einer Schichtdicke von weniger als 50 µm, vorzugsweise etwa 10 µm aufgebracht wird.

11. Verfahren nach einem der Ansprüche 5 bis 10, **dadurch gekennzeichnet, dass** das Hilfsband (24) vorzugsweise durch ein Koextrusionsverfahren über seine Länge mit Heißklebemittel beschichtet wird.

12. Verfahren nach einem der Ansprüche 5 bis 11, **dadurch gekennzeichnet, dass** der Bandendabschnitt über einen Umschlingungsbereich von weniger als 180°, vorzugsweise weniger als 90° fest mit der Spule (14,16) verbunden wird.

13. Verfahren nach Anspruch 5, **dadurch gekennzeichnet, dass** die kraftschlüssige Bandverbindung (32) durch eine Klemmverbindung zwischen dem Bandendabschnitt und der zugeordneten Spule (14,16) hergestellt wird.

14. Verfahren nach Anspruch 13, **dadurch gekennzeichnet, dass** der Bandendabschnitt in einem Klemmspalt der Spule (14,16) eingeklemmt wird.

## Claims

1. Process for producing a diagnostic tape cassette (10) for carrying out blood sugar tests in which disposable test units (20) for blood sugar tests, in the form of analytical elements, are applied to a rollable transport tape (18), and in which the transport tape (18) is wound onto an unwinding spool (14) and is connected to a take-up spool (16) such that the test units (20) can be successively made available to a user by winding on the transport tape (18), and in which the leading end of the transport tape (18) is connected to the take-up spool (16) by means of an auxiliary tape (24), **characterized in that** the test units (20) are examined for rejects and a corresponding part of the transport tape (18) containing defective material is cut out, and thereafter the remaining sections of the transport tape are joined together at a connecting site (36) by means of a piece of adhesive tape (34), and that the trailing end of the transport tape (18) is connected by means of the auxiliary tape (24) to the unwinding spool (14).

2. Process according to claim 1, **characterized in that** the transport tape (18) is butt-joined to the auxiliary tape (24) by means of a piece of adhesive tape (34).

3. Process according to claim 1, **characterized in that** the transport tape (18) is joined to the auxiliary tape (24) in an overlapping manner.

4. Process according to claim 3, **characterized in that** the transport tape (18) and the auxiliary tape (24) are materially joined together in an overlapping region at their ends and are preferably glued together by a layer of hot-melt adhesive (30) on the auxiliary tape (24).

5. Process according to one of the claims 1 to 4, **characterized in that** a free tape end section of the transport tape (18) or of an auxiliary tape (24) connected thereto is firmly connected to the unwinding spool (14) and/or take-up spool (16) by means of a material or frictional tape connection, such that the tape end section does not self-detach when it unwinds.

6. Process according to claim 5, **characterized in that** the material tape connection (32) is made by welding or gluing.

7. Process according to claim 5 or 6, **characterized in that** the material tape connection (32) is made by a hot-melt adhesive.

8. Process according to one of the claims 5 to 7, **characterized in that** the tape end section and/or the spool that is to be connected thereto is coated as a connecting partner with a hot-melt adhesive in a preparatory step and subsequently the connecting partners are joined together in a planar fashion by applying pressure and heat preferably by means of a welding die.

9. Process according to one of the claims 5 to 8, **characterized in that** the hot-melt adhesive is applied in a wet form and subsequently dried so that a portion of solids remains as the adhesive layer (30).

10. Process according to one of the claims 5 to 9, **characterized in that** an adhesive layer (30) with a layer thickness of less than 50 µm, preferably of about 10 µm is applied.

11. Process according to one of the claims 5 to 10, **characterized in that** the auxiliary tape (24) is coated with hot-melt adhesive over its entire length preferably by means of a coextrusion process.

12. Process according to one of the claims 5 to 11, **characterized in that** the tape end section is firmly connected to the spool (14, 16) over a wrap region of less than 180°, preferably of less than 90°.

13. Process according to claim 5, **characterized in that** the frictional tape connection (32) is made by a clamp connection between the tape end section and the associated spool (14, 16).

14. Process according to claim 13, **characterized in that** the tape end section is clamped in a clamping gap of the spools (14, 16).

## Revendications

1. Procédé pour la fabrication d'une cassette diagnostique à bande (10) pour la mise en oeuvre de contrôles de la glycémie (10), dans lequel des unités de tests jetables (20) réalisées sous la forme d'éléments d'analyse sont appliquées pour des contrôles de la glycémie sur une bande transporteuse (18) apte à s'enrouler, et dans lequel la bande transporteuse (18) vient s'enrouler sur une bobine de dévidage (14) et est reliée à une bobine d'enroulement (16), tant et si bien que les unités de tests (20) sont disponibles de manière successive pour un utilisateur via une avance rapide de la bande transporteuse (18), et dans lequel la bande transporteuse (18) est reliée via une bande auxiliaire (24) à son extrémité amont à la bobine d'enroulement (16), **caractérisé en ce que** les unités de tests (20) font l'objet d'une analyse en ce qui concerne le rebut et une partie correspondante de la bande transporteuse (18) contenant une mauvaise matière est découpée, et ensuite, les tronçons subsistants de la bande transporteuse sont reconstitués à un endroit de liaison (36) au moyen d'un morceau de bande adhésive (34), et **en ce que** la bande transporteuse (18) est reliée via la bande auxiliaire (24) à son extrémité aval à la bobine de dévidage (14).

2. Procédé selon la revendication 1, **caractérisé en ce que** la bande transporteuse (18) est reliée bout à bout via un morceau de bande adhésive (34) à la bande auxiliaire (24).

3. Procédé selon la revendication 1, **caractérisé en ce que** la bande transporteuse (18) est reliée en chevauchement à la bande auxiliaire (24).

4. Procédé selon la revendication 3, **caractérisé en ce que** la bande transporteuse (18) et la bande auxiliaire (24) sont reliées l'une à l'autre par adhérence dans une zone de chevauchement du côté terminal, de préférence sont collées via une couche thermoadhésive (30) de la bande auxiliaire (24).

5. Procédé selon l'une quelconque des revendications 1 à 4, **caractérisé en ce que** la bande transporteuse (18) ou une bande auxiliaire (24) qui lui est reliée, est reliée à demeure à un tronçon terminal libre de bande via une liaison de bande (32) par adhérence ou par friction à la bobine de dévidage (14) et/ou à la bobine d'enroulement (16), tant et si bien que le tronçon terminal de bande, lors du dévidage, ne se détache pas de manière automatique.

6. Procédé selon la revendication 5, **caractérisé en ce que** la liaison de bande (32) par adhérence est mise en oeuvre par soudure ou par collage.

7. Procédé selon la revendication 5 ou 6, **caractérisé en ce que** la liaison de bande (32) par adhérence est mise en oeuvre via un agent thermoadhésif.

8. Procédé selon l'une quelconque des revendications 5 à 7, **caractérisé en ce que,** au cours d'une étape préparatoire, le tronçon terminal de bande et/ou la bobine qui doit venir s'y relier à titre de partenaire de liaison sont enduits d'un agent thermoadhésif, et **en ce que** par la suite, les partenaires de liaison sont reliés l'un à l'autre sur toute leur surface par l'application de pression et de chaleur, de préférence au moyen d'un tampon de soudage.

9. Procédé selon l'une quelconque des revendications 5 à 8, **caractérisé en ce que** l'agent thermoadhésif est appliqué à l'état humide et est ensuite séché, d'une manière telle qu'une fraction de matière solide subsiste à titre de couche adhésive (30).

10. Procédé selon l'une quelconque des revendications 5 à 9, **caractérisé en ce qu'on** applique une couche adhésive (30) dont l'épaisseur de couche est inférieure à 50 µm, de préférence s'élève à environ 10 µm.

11. Procédé selon l'une quelconque des revendications 5 à 10, **caractérisé en ce qu'on** enduit la bande auxiliaire (24) avec un agent thermoadhésif sur sa longueur, de préférence via un procédé de coextrusion.

12. Procédé selon l'une quelconque des revendications 5 à 11, **caractérisé en ce que** le tronçon terminal de bande est relié à demeure à la bobine (14, 16) via une zone d'enroulement inférieure à 180°, de préférence inférieure à 90°.

13. Procédé selon la revendication 5, **caractérisé en ce que** la liaison de bande (32) par friction est mise en oeuvre via une liaison par serrage entre le tronçon terminal de bande et la bobine correspondante (14, 16).

14. Procédé selon la revendication 13, **caractérisé en ce que** le tronçon terminal de bande est enserré dans une fente de serrage de la bobine (14, 16).
